# EUROPEAN PATENT APPLICATION

(11) **EP 4 830 716 A1**
(43) Date of publication of application: **28.10.2026**
(21) Application number: 24908375.9
(22) Date of filing: 19.12.2024
(51) Int. Cl.: A61K 38/17, A61P 3/04

(54) **PHARMACEUTICAL COMPOSITION COMPRISING EFPEGLENATIDE AS ACTIVE INGREDIENT AND ADMINISTRATION THERAPY THEREOF**

(30) Priority: 19.12.2023 KR 20230186003
(71) Applicant: Hanmi Pharm. Co., Ltd., Hwaseong-si, Gyeonggi-do 18536 (KR)
(72) Inventor: KIM, Nayoung, Hwaseong-si Gyeonggi-do 18536 (KR); HONG, Sunghee, Hwaseong-si Gyeonggi-do Hwaseong-si (KR); KWACK, Hoyoung, Hwaseong-si Gyeonggi-do 18536 (KR); LIM, Yirang, Hwaseong-si Gyeonggi-do 18536 (KR); KIM, Jung-Kuk, Hwaseong-si Gyeonggi-do 18536 (KR); BAEK, Eunhye, Hwaseong-si Gyeonggi-do 18536 (KR)
(74) Representative: Mewburn Ellis LLP
(86) International application number: PCT/KR2024/097176
(87) International publication number: WO 2025/136071

(57) **Abstract**

Provided are a pharmaceutical composition comprising efpeglenatide as an active ingredient, and a method of treating obesity using the same. The administration of the composition comprising efpeglenatide according to the present disclosure may exhibit a weight loss effect on obese patients without side effects.

## Description

### [Technical Field]

The present disclosure relates to a pharmaceutical composition comprising efpeglenatide as an active ingredient, and a method of preventing and treating obesity using the same.

### [Background Art]

Obesity is a metabolic disease caused by an energy imbalance when nutrient intake exceeds energy expenditure over a long period of time, and it is also caused by a complex set of genetic or phenotypic abnormalities of various neuroendocrine substances and multiple factors involved in energy metabolism. The diagnosis of obesity requires a comprehensive assessment of body mass index (BMI), bioimpedance analysis (BIA), waist circumference, and abdominal fat CT scans, etc., but the body mass index, which can be easily estimated using only height and weight, is generally used as the criterion for diagnosing obesity and classifying stage thereof.

The World Health Organization classifies a body mass index of less than 25 kg/m² as normal, a range of 25 kg/m² to 29.99 kg/m² as overweight, and 30 kg/m² or higher as obesity, and most countries use these criteria. However, since the relationship between body mass index or body fat percentage and disease morbidity or mortality varies by race and ethnicity, there has been a need to establish criteria by taking into account racial and ethnic characteristics rather than defining obesity uniformly. Accordingly, some East Asian countries use slightly different criteria. In Korea, obesity is diagnosed and classified based on the criteria of a body mass index of less than 25 kg/m² as normal, 25 kg/m² - 29.99 kg/m² as obesity stage 1, and 30 kg/m² or higher as obesity stage 2 or 3, rather than the World Health Organization's criteria.

Currently, the basic treatment for obesity is dietary therapy, exercise therapy, and behavioral therapy, and it is recommended to use medication as an additional treatment method in combination therewith. Recently, a new market has been formed centered on GLP-1 agents as therapeutic agents for obesity.

Glucagon-Like Peptide-1 (GLP-1) is an incretin hormone secreted by L cells in the ileum and colon, and was first discovered in 1985. When blood glucose exceeds the basal level (4.5 mM) after food intake, GLP-1 binds to receptors on pancreatic β cells to stimulate insulin secretion, thereby lowering blood glucose. It has a characteristic of stimulating insulin secretion depending on blood glucose concentrations, and accordingly, hypoglycemia does not occur. Further, since GLP-1 suppresses glucagon secretion, it has been approved and marketed worldwide, including in Korea, as a therapeutic agent for type 2 diabetes mellitus (T2DM). Afterwards, as GLP-1's effects of lowering the upper digestive tract motility, delaying stomach emptying, and suppressing appetite were further identified, its development as a therapeutic agent for obesity was further advanced, and Liraglutide and Semaglutide, which are GLP-1 analogues, as therapeutic agents for obesity, are being used worldwide. However, most GLP-1 analogue preparations to date are for obese patients with a body mass index of 30 kg/m² or higher, or a body mass index of 27 kg/m² or higher and one or more comorbidities according to the World Health Organization's obesity diagnostic criteria, and there are no therapeutic agents that can also be used for obese patients with a body mass index of 25 kg/m² to 29.99 kg/m².

On the other hand, even though the drug's efficacy is demonstrated based on in-vitro experiments, when administered to an individual (particularly, a human), it is necessary to determine the dosing regimens and doses exerting efficacy while being safe through clinical trials, which is why it is difficult to develop a drug that can be commercialized by passing through practical clinical trials.

Since dose translation from animal to human studies is very complex and difficult, it is presented as one of the representative difficulties faced in new drug development (Reagan-Shaw S et al., Dose translation from animal to human studies revisited. Fed Am Soc Exp Biol J 2008). In other words, this means that the optimal administration cycle and dosage can be derived only through clinical trial design and results that consider all aspects of pharmacological action, side effects, and tolerance dose (dosage that can be safely administered) in the human body.

In addition, when determining the administration cycle, it is important to consider the drug's duration, which is often predicted through the half-life. In other words, when the duration of a drug's effective dose is 6 hours, it is effective to administer the drug 4 times a day. Although the drug's efficacy usually increases as the dose increases, side effects also increase, and therefore, it is not desirable to increase the dose indefinitely. In addition, most drugs tend to peak its maximum efficacy when reaches a certain dosage, and the efficacy does not further increase (sigmoidal curve), and rather, the drug's efficacy decreases (biphasic effect) in some cases. Therefore, when determining the administration cycle and dosage, all of these factors must be considered together, and thus it is by no means an easy process.

In this aspect, even for a drug whose efficacy is already known, if it is possible to increase its therapeutic efficacy, to reduce side effects, and to increase convenience of taking the drug by using dosing regimens different from the dosing regimens previously used, this has value no less than that of developing a new pharmaceutical substance or medicinal use.

### [Disclosure]

### [Technical Problem]

When administering efpeglenatide, it is necessary to identify a clinically effective dosing regimen and dosage that may maintain usefulness of the drug while avoiding critical side effects.

### [Technical Solution]

An object of the present disclosure is to provide a pharmaceutical composition for preventing or treating obesity, the pharmaceutical composition comprising efpeglenatide as an active ingredient, wherein 8 mg to 18 mg of efpeglenatide is parenterally administered to obese patients once a week.

### [Advantageous Effects]

A composition comprising efpeglenatide according to the present disclosure may be applied to the treatment of obesity without side effects while ensuring the convenience of administration to patients with once-weekly administration.

### [Brief Description of the Drawing]

FIG. 1 shows changes in the body weight by efpeglenatide in animal models, in which (A) shows changes in the body weight over the entire period (12 weeks), and (B) and (C) show changes in the body weight (~Eₘₐₓ) on day 16 (D16) and day 86 (D86, the end of the test), respectively (***p<0.001 vs. AMLN, vehicle by One-way ANOVA test);
FIG. 2 shows the results of estimating the weight loss rate, relative to placebo, when administered repeatedly once a week at each dose level (0.5 mg, 1 mg, 2 mg, 3 mg, 4 mg, 5 mg, 6 mg, 7 mg, 8 mg, 9 mg, 10 mg, 20 mg, 50 mg, 100 mg) through Population PK/PD simulation;
FIGS. 3A and 3B show the results of estimating the weight loss rate according to the time point at each dose level (3A target dose: 2 mg, 4 mg, 6 mg, 10 mg, 14 mg, 18 mg QW / 3B target dose: 2 mg, 4 mg, 6 mg, 8 mg, 10 mg, 12 mg, 14 mg, 16 mg, 18 mg QW) when dose titration was performed through weight loss simulation;
FIG. 4 shows the results of pharmacokinetic (PK) simulation according to dose titration up to 63 weeks, Case 1(①): 2-2-4-4-6-6-8-8-10mg QW, Case 2(②):2-2-4-4-6mg QW, Case 4(④): 2-2-4-4-6-6-6-6-8-8-8-8-10mg QW (Slow Titration);
FIGS. 5A to 5D show the cumulative probability of adverse reactions (nausea and vomiting) at each dose level when there was no dose titration period;
FIG. 6 shows the simulation results of the cumulative probability of adverse reactions of the gastrointestinal tract (nausea, vomiting) when 10 mg was administered after dose titration; and
FIG. 7 shows a schematic illustration of a clinical trial procedure of efpeglenatide.

### [Detailed Description of Preferred Embodiments]

An aspect for implementing the present disclosure provides a composition comprising efpeglenatide as an active ingredient.

In one specific embodiment, a pharmaceutical composition for preventing or treating obesity is characterized in that 8 mg to 18 mg of efpeglenatide is parenterally administered to obese patients once a week.

In another specific embodiment, a pharmaceutical composition for preventing or treating obesity is characterized in that 8 mg to 14 mg of efpeglenatide is parenterally administered to obese patients once a week.

The pharmaceutical composition according to any one of the previous specific embodiments is characterized in that 8 mg to 10 mg of efpeglenatide is parenterally administered to obese patients once a week.

The pharmaceutical composition according to any one of the previous specific embodiments is characterized in that the obese patients have a body mass index (BMI) of 25 kg/m² or higher.

The pharmaceutical composition according to any one of the previous specific embodiments is characterized in that the obese patients do not have diabetes.

The pharmaceutical composition according to any one of the previous specific embodiments is characterized in that the obese patients have
(a) a body mass index (BMI) of 30 kg/m² or higher, or
(b) a BMI of 27 kg/m² or higher, and lower than 30 kg/m² while having at least one or more risk factors or comorbidities.

The pharmaceutical composition according to any one of the previous specific embodiments is characterized in that the patients having risk factors or comorbidities are patients having any one or more diseases of hypertension, dyslipidemia, sleep apnea, and cardio-cerebrovascular diseases.

The pharmaceutical composition according to any one of the previous specific embodiments is characterized in that the patients having risk factors or comorbidities are patients selected from the following (a) to (d):
(a) patients with hypertension: taking medication for hypertension or having sitSBP ≥ 140 mmHg or sitDBP ≥ 90 mmHg
(b) patients with dyslipidemia: taking medication for dyslipidemia or having total cholesterol ≥ 240mg/dL or LDL-C ≥ 160mg/dL or TG ≥ 200 mg/dL or HDL-C < 40 mg/dL
(c) patients with sleep apnea
(d) patients with cardio-cerebrovascular diseases: ischemic cardio-cerebrovascular diseases, heart failure of NYHA Class I~III, etc.

The pharmaceutical composition according to any one of the previous specific embodiments is characterized in that the obese patients have undergone a dose titration period in which 2 mg to 8 mg of efpeglenatide is administered for 2 weeks to 12 weeks.

The pharmaceutical composition according to any one of the previous specific embodiments is characterized in that the dose titration is performed by administering efpeglenatide with dose increments of 2 mg or 4 mg over 1 to 4 times.

The pharmaceutical composition according to any one of the previous specific embodiments is characterized in that the obese patients have undergone a dose titration period in which 2 mg to 14 mg of efpeglenatide is administered for 2 weeks to 14 weeks.

The pharmaceutical composition according to any one of the previous specific embodiments is characterized in that the dose titration starts with administering 2 mg of efpeglenatide once a week for 2 weeks, and with sequentially administering efpeglenatide with dose increments of 2 mg or 4 mg once a week at 2-week or 4-week intervals to reach the final dose.

The pharmaceutical composition according to any one of the previous specific embodiments is characterized in that the dose titration is performed by sequentially administering 2 mg of efpeglenatide once a week for 2 weeks, 4 mg of efpeglenatide once a week for 2 weeks, 6 mg of efpeglenatide once a week for 4 weeks, and 8 mg of efpeglenatide once a week for 4 weeks.

The pharmaceutical composition according to any one of the previous specific embodiments is characterized in that the dose titration is performed by sequentially administering 2 mg of efpeglenatide once a week for 2 weeks, 4 mg of efpeglenatide once a week for 2 weeks, 6 mg of efpeglenatide once a week for 4 weeks, 10 mg of efpeglenatide once a week for 4 weeks, and 14 mg of efpeglenatide once a week for 4 weeks.

The pharmaceutical composition according to any one of the previous specific embodiments is characterized in that the dose titration is performed by sequentially administering 2 mg or more and less than 6 mg of efpeglenatide once a week for 2 weeks, and 6 mg or more and 16 mg or less of efpeglenatide once a week for 4 weeks.

The pharmaceutical composition according to any one of the previous specific embodiments is characterized in that the pharmaceutical composition is used in dosing regimens for preventing or treating obesity.

The pharmaceutical composition according to any one of the previous specific embodiments is characterized in that the pharmaceutical composition is administered to the arm, thigh, or abdomen.

The pharmaceutical composition according to any one of the previous specific embodiments is characterized in that the pharmaceutical composition is subcutaneously administered.

Another aspect of the present disclosure provides a method of preventing or treating obesity, the method comprising administering the composition comprising efpeglenatide as an active ingredient to obese patients.

In one specific embodiment, the method is characterized in that 8 mg to 18 mg of efpeglenatide is parenterally administered to obese patients once a week.

In another specific embodiment, the method is characterized in that 8 mg to 14 mg of efpeglenatide is parenterally administered to obese patients once a week.

In still another specific embodiment, the method is characterized in that 10 mg of efpeglenatide is parenterally administered to obese patients once a week.

The method according to any one of the previous specific embodiments is characterized in that the obese patients have a body mass index (BMI) of 25 kg/m² or higher.

The pharmaceutical composition according to any one of the previous specific embodiments is characterized in that the obese patients do not have diabetes.

The method according to any one of the previous specific embodiments is characterized in that the obese patients have
(a) a body mass index (BMI) of 30 kg/m² or higher, or
(b) a BMI of 27 kg/m² or higher, and lower than 30 kg/m² while having at least one or more risk factors or comorbidities.

The method according to any one of the previous specific embodiments is characterized in that the patients having risk factors or comorbidities have any one or more diseases of hypertension, dyslipidemia, sleep apnea, and cardio-cerebrovascular diseases.

The method according to any one of the previous specific embodiments is characterized in that the patients having risk factors or comorbidities are patients selected from the following (a) to (d):
(a) patients with hypertension: taking medication for hypertension or having sitSBP ≥ 140 mmHg or sitDBP ≥ 90 mmHg
(b) patients with dyslipidemia: taking medication for dyslipidemia or having total cholesterol ≥ 240mg/dL or LDL-C ≥ 160mg/dL or TG ≥ 200 mg/dL or HDL-C < 40 mg/dL
(c) patients with sleep apnea
(d) patients with cardio-cerebrovascular diseases: ischemic cardio-cerebrovascular diseases, heart failure of NYHA Class I~III, etc.

The method according to any one of the previous specific embodiments is characterized in that the method comprises a dose titration step of administering 2 mg to 14 mg of efpeglenatide to obese patients for 2 weeks to 14 weeks prior to the administration of 8 mg to 18 mg of efpeglenatide.

The method according to any one of the previous specific embodiments is characterized in that the method comprises a dose titration step of administering 2 mg to 8 mg of efpeglenatide to obese patients for 2 weeks to 12 weeks prior to the administration of 8 mg to 14 mg of efpeglenatide.

The method according to any one of the previous specific embodiments is characterized in that the dose titration step is performed by administering efpeglenatide with dose increments of 2 mg or 4 mg over 1 to 4 times.

The method according to any one of the previous specific embodiments is characterized in that the dose titration starts with administering 2 mg of efpeglenatide once a week for 2 weeks, and with sequentially administering efpeglenatide once a week with dose increments of 2 mg or 4 mg at 2-week or 4-week intervals to reach the final dose.

The method according to any one of the previous specific embodiments is characterized in that the dose titration step is performed by sequentially administering 2 mg of efpeglenatide once a week for 2 weeks, 4 mg of efpeglenatide once a week for 2 weeks, 6 mg of efpeglenatide once a week for 2 weeks or 4 weeks, 10 mg of efpeglenatide once a week for 4 weeks, and 14 mg of efpeglenatide once a week for 4 weeks.

The method according to any one of the previous specific embodiments is characterized in that the dose titration step is performed by sequentially administering 2 mg of efpeglenatide once a week for 2 weeks, 4 mg of efpeglenatide once a week for 2 weeks, 6 mg of efpeglenatide once a week for 4 weeks, and 8 mg of efpeglenatide once a week for 4 weeks.

The method according to any one of the previous specific embodiments is characterized in that the dose titration is performed by sequentially administering 2 mg or more and less than 6 mg of efpeglenatide once a week for 2 weeks, and 6 mg or more and 16 mg or less of efpeglenatide once a week for 4 weeks.

The method according to any one of the previous specific embodiments is characterized in that efpeglenatide is administered to the arm, thigh, or abdomen of obese patients.

The method according to any one of the previous specific embodiments is characterized in that the efpeglenatide is subcutaneously administered.

Still another aspect of the present disclosure provides use of the composition comprising efpeglenatide as an active ingredient in preventing or treating obesity.

In one specific embodiment, the efpeglenatide is characterized in that 8 mg to 18 mg thereof is parenterally administered to obese patients once a week.

In another specific embodiment, the efpeglenatide is characterized in that 8 mg to 14 mg thereof is parenterally administered to obese patients once a week. The use according to any one of the previous specific embodiments is characterized in that 8 mg to 10 mg of efpeglenatide is parenterally administered to obese patients once a week.

The use according to any one of the previous specific embodiments is characterized in that the obese patients have a body mass index (BMI) of 25 kg/m² or higher.

The pharmaceutical composition according to any one of the previous specific embodiments is characterized in that the obese patients do not have diabetes.

The use according to any one of the previous specific embodiments is characterized in that the efpeglenatide is subcutaneously administered.

### [Mode for Carrying Out the Invention]

Hereinafter, the present disclosure will be described in detail.

Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description provided below.

The disclosures of the cited papers and patent documents are incorporated herein by reference in their entirety.

Throughout the entire specification of the present disclosure, not only the conventional one-letter and three-letter codes for naturally occurring amino acids, but also three-letter codes generally allowed for other amino acids, such as 2-Aminoisobutyric acid (Aib), N-methylglycine (Sar), α-methyl-glutamic acid, etc. are used. Further, the amino acids abbreviated herein are described according to the nomenclature rules of IUPAC-IUB.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

An aspect to achieve the present disclosure provides a pharmaceutical composition for preventing or treating obesity, the pharmaceutical composition comprising efpeglenatide as an active ingredient, characterized in that 8 mg to 18 mg of the efpeglenatide is parenterally administered to obese patients once a week.

The pharmaceutical composition according to the present disclosure has technical significance in that it may be actually applied to the human body by identifying a specific administration dose and administration cycle capable of exhibiting safe and effective efficacy.

Efpeglenatide is a long-acting CA-exendin-4 derivative, and studies have been continuously conducted to develop therapeutic agents for diabetes and obesity using the same (WO 2008/082274 A1, WO 2009/011544 A2). The present disclosure has identified a method of administering efpeglenatide in high doses of 8 mg to 18 mg through a dose titration process to reduce the risk of side effects.

In a specific embodiment of the present disclosure, the pharmaceutical composition of the present disclosure comprises efpeglenatide as an active ingredient, which may be used in dosing regimens for preventing or treating obesity. Specifically, the pharmaceutical composition may comprise, but is not limited to, a pharmacologically effective amount of efpeglenatide and a pharmaceutically acceptable excipient.

The efpeglenatide of the present disclosure is a conjugate of an exendin-4 analogue (CA exendin-4) and a human-derived immunoglobulin IgG4 Fc region linked via 3.4 kDa of a propionaldehyde polyethylene glycol linker molecule (CAS No. 1296200-77-5).

CA exendin-4 is composed of 39 amino acids, and is an exendin-4 analogue in which a first residue is substituted with (imidazol-4-yl)acetic acid (SEQ ID NO: 1).

The human-derived immunoglobulin IgG4 Fc region is a homodimer (SEQ ID NO: 3) which is formed by a monomer (SEQ ID NO: 2) consisting of 221 amino acids through a disulfide bond between cysteines at position 3, and has a molecular weight of 49.8 kDa. Each monomer comprises two inter-disulfide bonds; specifically, disulfide bonds exist between cysteines at positions 35 and 95, and 141 and 199.

The binding of CA Exendin-4 to the immunoglobulin IgG4 Fc region occurs by the formation of an amine bond between aldehyde polyethylene glycol (OHC-CH₂CH₂O(CH₂CH₂O)ₙCH₂CH₂-CHO (n = 54 ~ 77)) and lysine at position 27 of CA Exendin-4 and the N-terminal amino acid group of the immunoglobulin IgG4 Fc region.

The safety of efpeglenatide of the present disclosure has been demonstrated in patients with type 2 diabetes or obese patients without type 2 diabetes through phase 2 clinical trials (NCT01452451, NCT02057172, NCT02081118, NCT02075281), etc., and it has been also demonstrated that the desired effect may be achieved when administered up to 6 mg once every 1 week or 8 mg once every 2 weeks.

However, in order to obtain a more effective weight loss effect, it was intended to investigate a high dose of efpeglenatide, of which safety is ensured, while also investigating an administration method of reducing side effects such as vomiting, nausea, gastrointestinal adverse reactions, etc., which commonly occur by GLP-1 analog drugs. As a result, it was confirmed that such side effects may be minimized through a dose titration period while administering 8 mg to 18 mg of high-dose efpeglenatide.

The efpeglenatide of the present disclosure may be administered to obese patients in a pharmacologically effective amount.

In the present disclosure, the term "pharmacologically effective amount" refers to a safe dosage of efpeglenatide exhibiting a therapeutic effect on obese patients without causing toxicity or side effects in patients. Specifically, it may refer to a dosage that may exhibit effects such as weight loss, BMI reduction, waist circumference reduction, blood lipid reduction, etc. in obese patients, but is not limited thereto.

In a specific embodiment of the present disclosure, the efpeglenatide according to the present disclosure may be administered at a single dose of about 8 mg to about 18 mg, about 10 mg to about 18 mg, about 8 mg to about 14 mg, about 9 mg to about 14 mg, about 10 mg to about 14 mg, or about 10 mg to about 12 mg, specifically, 8.0 mg, 8.1 mg, 8.2 mg, 8.3 mg, 8.4 mg, 8.5 mg, 8.6 mg, 8.7 mg, 8.8 mg, 8.9 mg, 9.0 mg, 9.1 mg, 9.2 mg, 9.3 mg, 9.4 mg, 9.5 mg, 9.6 mg, 9.7 mg, 9.8 mg, 9.9 mg, or 10.0 mg or more, and about 18 mg, 17.9 mg, 17.8 mg, 17.7 mg, 17.6 mg, 17.5 mg, 17.4 mg, 17.3 mg, 17.2 mg, 17.1 mg, 17.0 mg, 16.9 mg, 16.8 mg, 16.7 mg, 16.6 mg, 16.5 mg, 16.4 mg, 16.3 mg, 16.2 mg, 16.1 mg, or 16.0 mg or less, but is not limited thereto.

Specifically, the long-acting conjugate of a triple activator of the present disclosure may be administered at a single dose of about 8 mg to about 18 mg, about 8 mg to about 14 mg, about 8 mg to about 12 mg, about 8.0 mg, about 9.0 mg, about 10.0 mg, about 11.0 mg, about 12.0 mg, about 13.0 mg, about 14.0 mg, about 15.0 mg, about 16.0 mg, about 17.0 mg, or about 18.0 mg, but is not limited thereto, and the above single dose may be appropriately adjusted by a doctor or prescriber, based on the severity of the disease, the patient's age, the administration period, etc. The single dose refers to a dose that must be administered at regular intervals for obesity treatment.

The efpeglenatide may be administered at a constant dose regardless of the patient's body weight, but it may be appropriately adjusted according to the body weight, as needed.

As used herein, the term "about" refers to a range which comprises all of ±0.5, ±0.4, ±0.3, ±0.2, ±0.1, ±0.05, ±0.01, etc. and comprises all of the values that are equivalent or similar to those following the values, but the range is not limited thereto.

The efpeglenatide of the present disclosure may be administered at a single dose described above for a predetermined period of time at regular intervals, thereby exhibiting a prophylactic or therapeutic effect on obesity, but is not limited thereto.

The pharmaceutical composition of the present disclosure may be a composition comprising efpeglenatide, and may be used in dosing regimens for preventing or treating obesity. Specifically, the pharmaceutical composition of the present disclosure may comprise efpeglenatide in the single dose described above, and may further comprise a pharmaceutically acceptable excipient in a required amount, but is not limited thereto.

The efpeglenatide of the present disclosure has an extended half-life due to its binding to the immunoglobulin Fc region, so that it exerts its pharmacological effect for a sufficient period. As a result, the dosing intervalcan be extended, thereby improving patient convenience.

Specifically, the efpeglenatide of the present disclosure may be administered once a week, once every two weeks, once every three weeks, once every four weeks, or once every month, but is not particularly limited thereto as long as it is an administration cycle that may maintain a body concentration capable of exerting its pharmacological effect.

The efpeglenatide of the present disclosure may be administered to patients through a fractionated treatment protocol in which multiple doses are administered over an extended period.

With respect to the objects of the present disclosure, the pharmaceutical composition of the present disclosure may be administered for a period of time sufficient to exhibit the therapeutic effect. For example, the period may be a period of time sufficient to return one or more of the abnormal indicators of obese patients to a normal range recognized in the art (e.g., BMI of lower than 23), such as weight loss, BMI reduction, waist circumference reduction, blood lipid reduction, blood pressure reduction, improvement in body composition (body fat mass, lean body mass, visceral fat mass), hepatic steatosis improvement, impaired glucose tolerance (IGT) improvement, etc., but is not limited thereto. For example, it may be administered for 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, 50 weeks, 51 weeks, 52 weeks, 53 weeks, 54 weeks, 55 weeks, 56 weeks, 57 weeks, 58 weeks, 59 weeks, 60 weeks, 61 weeks, 62 weeks, 63 weeks, or 64 weeks or longer, and the administration period may be appropriately adjusted depending on the severity of the disease.

The pharmaceutical composition according to the present disclosure may be administered once at one-week intervals during the above administration period.

The pharmaceutical composition according to the present disclosure may be administered to obese patients who have undergone a dose titration period.

In the present disclosure, the term "dose titration" refers to a process of administering a lower dose of efpeglenatide than the target dose of efpeglenatide at the beginning of administration and gradually increasing the dose of efpeglenatide to ultimately reach the target dose of efpeglenatide in order to reduce adverse reactions (such as vomiting, nausea, gastrointestinal adverse reactions, etc.) in patients, before administering a high concentration of efpeglenatide, and the dose titration period refers to a period during which the dose titration is performed.

The efpeglenatide administered during the dose titration may be 2 mg to 16 mg per administration, but is not limited thereto, and any dose lower than the final target dose may be used for dose titration. For a specific example, efpeglenatide administered during the dose titration may be, but is not limited to, about 2 mg, 3 mg, 4 mg, 5 mg, 6 mg, 7 mg, 8 mg, 9 mg, 10 mg, 11 mg, 12 mg, 13 mg, 14 mg, 15 mg, or 16 mg per administration.

Specifically, the pharmaceutical composition according to the present disclosure may be characterized in that it is administered to a patient who has undergone a dose titration of administering a low dose (e.g., 16 mg or less) of efpeglenatide for a predetermined period of time before administration of the target dose, in order to reduce side effects such as vomiting, nausea, gastrointestinal adverse reactions, etc., while administering 8 mg to 18 mg of high-dose efpeglenatide to increase the weight loss effect.

In this case, the low dose of efpeglenatide means a dose lower than the final target administration dose of efpeglenatide, and the increase in dosage during the dose titration may be 1 mg, 2 mg, 3 mg, or 4 mg, but is not limited thereto. The increased dose may be equal during the dose titration period, or may be changed (decreased or increased) during the titration period, but is not limited thereto. In addition, the dose titration period may be, but is not limited to, 1 week or longer, 20 weeks or shorter, 18 weeks or shorter, 16 weeks or shorter, 14 weeks or shorter, or 12 weeks or shorter, and may be determined as a sufficient time to reach the final target dose. For example, when the dose titration is performed using less than 6 mg of efpeglenatide, administration may be performed for 2 weeks, and when the dose titration is performed using 6 mg or more of efpeglenatide, administration may be performed for 2 weeks or 4 weeks, but is not limited thereto.

In addition, during the dose titration period, efpeglenatide for the dose titration may be administered once or more, twice or more, three times or more, four times or more, five times or more, or six or more times, but is not limited thereto.

In addition, during the dose titration period, efpeglenatide may be administered daily, once every two days, once a week, once every two weeks, once every three weeks, or once every four weeks, but is not limited to a specific administration cycle as long as it may reach the final target dose of efpeglenatide while reducing side effects.

In one embodiment, the obese patients may be patients who are administered 1 mg to 16 mg of efpeglenatide every week for 2 weeks to 20 weeks, but are not limited thereto, specifically, patients who are administered 2 mg to 16 mg, 2 mg to 14 mg, 2 mg to 12 mg, 2 mg to 10 mg, 2 mg to 8 mg of efpeglenatide for 2 weeks to 20 weeks, 2 weeks to 16 weeks, 2 weeks to 10 weeks, or 2 weeks to 8 weeks, but are not limited thereto.

In another embodiment, the obese patients may be patients who are administered 1 mg to 8 mg of efpeglenatide every week for 2 weeks to 12 weeks, but are not limited thereto.

Specifically, the obese patients may be patients who are administered 2 mg to 8 mg, 2 mg to 6 mg, 2 mg to 4 mg of efpeglenatide for 2 weeks to 12 weeks, 2 weeks to 10 weeks, 2 weeks to 8 weeks, but are not limited thereto.

In still another embodiment, the obese patients may be patients who are sequentially administered 2 mg of efpeglenatide once a week for 2 weeks, 4 mg of efpeglenatide once a week for 2 weeks, 6 mg of efpeglenatide once a week for 4 weeks, and 8 mg of efpeglenatide once a week for 4 weeks, but are not limited thereto.

In still another embodiment, when the target dose of efpeglenatide is 10 mg, the obese patients may be patients who are sequentially administered 2 mg of efpeglenatide once a week for 2 weeks, 4 mg of efpeglenatide once a week for 2 weeks, and 6 mg of efpeglenatide once a week for 2 weeks or 4 weeks, before administration of 10 mg of efpeglenatide, and after administration of 6 mg, additionally, administered 8 mg of efpeglenatide once a week for 2 weeks or 4 weeks depending on the patient's drug tolerance, but are not limited thereto.

In still another embodiment, when the target dose of efpeglenatide is 14 mg, the obese patients may be patients who are sequentially administered 2 mg of efpeglenatide once a week for 2 weeks, 4 mg of efpeglenatide once a week for 2 weeks, 6 mg of efpeglenatide once a week for 2 weeks or 4 weeks, and 10 mg of efpeglenatide once a week for 4 weeks, before administration of 14 mg of efpeglenatide, and after administration of 10 mg, additionally, administered 12 mg of efpeglenatide once a week for 2 weeks or 4 weeks depending on the patient's drug tolerance, but are not limited thereto.

In still another embodiment, when the target dose of efpeglenatide is 18 mg, the obese patients may be patients who are sequentially administered 2 mg of efpeglenatide once a week for 2 weeks, 4 mg of efpeglenatide once a week for 2 weeks, 6 mg of efpeglenatide once a week for 2 weeks or 4 weeks, 10 mg of efpeglenatide once a week for 4 weeks, and 12 mg of efpeglenatide once a week for 4 weeks, before administration of 18 mg of efpeglenatide, and after administration of 12 mg, additionally, administered 14 mg or 16 mg of efpeglenatide once a week for 2 weeks or 4 weeks depending on the patient's conditions, but are not limited thereto.

The pharmaceutical composition of the present disclosure may be administered to obese patients.

As used herein, the term "obesity" refers to a state of excessive fat tissue in the body, and obesity is usually caused by an energy imbalance when nutrient intake exceeds energy expenditure over a long period of time. Obesity is a metabolic disease that affects throughout the entire body, increases the possibility of diabetes and hyperlipidemia, and increases the risk of developing sexual dysfunction, arthritis, and cardiovascular disease, and in some cases, is also associated with the occurrence of cancer.

The World Health Organization (WHO) classifies a Body Mass Index(BMI) of lower than 25 kg/m² as normal, a range of 25 to 29.99 kg/m² as overweight, and 30 kg/m² or higher as obesity. However, a more detailed classification is necessary, taking into account racial and ethnic characteristics. In Korea, instead of the WHO criteria, obesity is diagnosed and classified based on the criteria of a BMI of lower than 25 kg/m² as normal, 25 to 29.99 kg/m² as obesity stage 1, and 30 kg/m² or higher as obesity stage 2 or 3.

Obese patients who are administered the pharmaceutical composition according to the present disclosure may be patients with a body mass index (BMI) of 25 kg/m² or higher, but are not limited thereto. Specifically, the obese patients may be patients with a BMI of 25 kg/m² or higher, 26 kg/m² or higher, 27 kg/m² or higher, 28 kg/m² or higher, 29 kg/m² or higher, or 30 kg/m² or higher, but are not limited thereto.

In addition, the obese patients of the present disclosure may be obese patients without diabetes, particularly, type 2 diabetes, but are not limited thereto.

As used herein, the "obese patients without diabetes" refer to obese patients who have not been diagnosed with diabetes, and specifically, may refer to, but are not limited to, patients who are not those with a blood glucose level of 200 mg/dL or higher when measured regardless of meal time, a fasting blood glucose level of 126 mg/dL or higher when measured in a fasting state without calorie intake for 8 hours, or a 2-hour blood glucose level of 200 mg/dL or higher when measured after ingesting 75 mg of glucose in an oral glucose tolerance test.

Alternatively, the obese patients without diabetes in the present disclosure may refer to obese patients whose glycated hemoglobin (HbA1c) level is less than 6.5%, but are not limited thereto.

Specifically, the obese patients who are administered the pharmaceutical composition according to the present disclosure may refer to patients having (a) a body mass index (BMI) of 30 kg/m² or higher, or (b) a BMI of 27 kg/m² or higher and lower than 30 kg/m² while having at least one or more risk factors or concomitant diseases, but are not limited thereto.

The patients with the above risk factors or concomitant diseases refer to patients with any one or more of hypertension, dyslipidemia, sleep apnea, and cardio-cerebrovascular diseases.

Specifically, the patients having hypertension, dyslipidemia, sleep apnea, or cardio-cerebrovascular diseases may be, but are not limited to, patients who meet the following criteria:
(a) patients with hypertension: taking medication for hypertension or having sitSBP ≥ 140 mmHg or sitDBP ≥ 90 mmHg
(b) patients with dyslipidemia: taking medication for dyslipidemia or having total cholesterol ≥ 240mg/dL or LDL-C ≥ 160mg/dL or TG ≥ 200 mg/dL or HDL-C < 40 mg/dL
(c) patients with sleep apnea
(d) patients with cardio-cerebrovascular diseases: ischemic cardio-cerebrovascular diseases, heart failure of NYHA Class I~III, etc.

In addition, the pharmaceutical composition of the present disclosure may be administered to patients who are diagnosed with obesity according to obesity diagnostic criteria known in the art, other than BMI.

Even though efpeglenatide of the present disclosure is a drug of the GLP-1 analogue series well known as a therapeutic agent for diabetes, it may be used for the treatment of obesity because it shows a weight loss effect on obese patients without diabetes, regardless of diabetes.

The pharmaceutical composition according to the present disclosure may show effects of weight loss, BMI reduction, waist circumference reduction, blood lipid reduction, blood pressure reduction, body composition (body fat mass, lean body mass, visceral fat mass) improvement, hepatic steatosis improvement, and impaired glucose tolerance (IGT) improvement in obese patients.

The dosage of the pharmaceutical composition of the present disclosure may be adjusted according to the severity of obesity and the administration period.

A formulation of the pharmaceutical composition of the present disclosure may be variously prepared by mixing it with pharmaceutically acceptable excipients. In a specific embodiment, the pharmaceutical composition of the present disclosure may be formulated into a formulation suitable for administering efpeglenatide, particularly, parenterally. In addition, the pharmaceutical composition of the present disclosure may be formulated into a stabilized formulation capable of maintaining pharmacological activity of efpeglenatide, but is not limited thereto.

For example, the pharmaceutical composition of the present disclosure may be prepared into a unit dosage ampoule or multiple dosage form. It may be formulated into a solution, suspension, tablet, pill, capsule, sustained-release preparation, etc.

Specifically, the pharmaceutical composition may be formulated into an injectable form so that it may be subcutaneously administered.

Injectable formulations are administered directly into the human body, either subcutaneously, blood vessels (blood injection), or muscles (intramuscular injection), and thus they do not undergo absorption in the gastrointestinal tract or metabolism in the liver. Therefore, solubility or absorption rate are not issues, and they may exert effects directly. However, since injectable formulations may directly affect human tissues, extreme care must be taken regarding toxicity due to drug components. In other words, the safety of the parenteral administration may be guaranteed only through the design of clinical trials that take into account the pharmacological action, side effects, tolerable dose (the dose to be safely administered) in the human body, etc., and the results thereof.

Further, the pharmaceutical composition of the present disclosure may be formulated into a unit dosage form suitable for administration into a patient's body according to conventional methods in the pharmaceutical field, specifically, into a formulation useful for administration of protein drugs, and then may be administered using a parenteral administration method commonly used in the art, and as the parenteral administration method, a person skilled in the art may appropriately select a route from skin, intravenous, intramuscular, intraarterial, intramedullary, intrathecal, intraventricular, pulmonary, transdermal, subcutaneous, intraperitoneal, intranasal, intragastric, topical, sublingual, intravaginal, or rectal route. Specifically, the pharmaceutical composition of the present disclosure may be administered by subcutaneous administration, and more specifically, the pharmaceutical composition of the present disclosure may be subcutaneously administered to the patient's arm (upper arm), thigh, or abdomen, but is not limited thereto.

With respect to the objects of the present disclosure, a subject who has been administered the pharmaceutical composition may exhibit one or more of the following characteristics, thereby having a prophylactic or therapeutic effect on obesity:
weight loss, BMI reduction,
waist circumference reduction,
blood lipid reduction, blood pressure reduction, body composition (body fat mass, lean body mass, visceral fat mass) improvement, hepatic steatosis improvement, impaired glucose tolerance improvement.

Specifically, a patient who has been administered the pharmaceutical composition of the present disclosure may have a weight loss of about 5% to about 15%, or at most 20% or more, 2 weeks after administration, as compared to the initial administration, but is not limited thereto.

The above description may also be applied to other specific embodiments or other aspects of the present disclosure, but is not limited thereto.

The pharmaceutical composition of the present disclosure may further comprise a pharmaceutically acceptable carrier, excipient, or diluent. Such a pharmaceutically acceptable carrier, excipient, or diluent may be non-naturally occurring.

As used herein, the term "pharmaceutically acceptable" means a sufficient amount to exhibit a therapeutic effect without causing side effects, and may be easily determined by those skilled in the art, depending on factors well known in the medical field, such as the type of disease, the patient's age, body weight, health, sex, the patient's drug sensitivity, the route of administration, the mode of administration, the number of administrations, the duration of treatment, and drugs used in combination or concurrently, etc.

The pharmaceutical composition of the present disclosure may further comprise a pharmaceutically acceptable carrier. The carrier may comprise, but is not particularly limited to, buffers, preservatives, analgesics, solubilizers, isotonic agents, stabilizers, etc. in a mixture thereof.

The pharmaceutical composition of the present disclosure is characterized in that it comprises efpeglenatide in a pharmacologically effective amount of, specifically, 8 mg to 18 mg, 8 mg to 14 mg, 8 mg to 10 mg, 10 mg to 18 mg, 12 mg to 18 mg, or 10 mg to 14 mg, and is administered to obese patients once a week.

This once-weekly administration of 8 mg to 18 mg, 8 mg to 14 mg, 8 mg to 10 mg, 10 mg to 18 mg, 12 mg to 18 mg, or 10 mg to 14 mg has a technical significance in that it is the optimal administration cycle and administration dose capable of exhibiting efficacy and reducing drug tolerance as well as side effects through practical clinical trials targeting obese patients, and has a practical prophylactic or therapeutic effect on obesity by administering the pharmaceutical composition according to the present disclosure.

Still another aspect of the present disclosure provides a method of preventing or treating obesity, the method comprising the step of administering the pharmaceutical composition comprising efpeglenatide to obese patients.

The method of preventing or treating obesity of the present disclosure may be to administer the pharmaceutical composition according to the above-described dosing regimen and dosage.

Specifically, the method of preventing or treating obesity may be to administer 8 mg to 18 mg of efpeglenatide parenterally (in particular, subcutaneously) to obese patients once a week, but is not limited thereto.

The obese patients may be patients who have undergone the dose titration period, but are not limited thereto.

In another specific embodiment, the method of preventing or treating obesity may further comprise the dose titration step before the step of administering 8 mg to 18 mg of efpeglenatide, but is not limited thereto.

As used herein, the term "dose titration step" refers to a step of performing the above-described dose titration, which involves administering a lower dose of efpeglenatide than the target dose at the beginning of administration and gradually increasing it until the target dose is ultimately reached.

The dose increment may be, but is not limited to, 1 mg, 2 mg, 3 mg, or 4 mg, but is not limited thereto, and the dose increment may be equal during the dose titration step, or may be changed (decreased or increased) during the dose titration step, but is not limited thereto. In addition, the dose titration step may be performed for, but is not limited to, 20 weeks or shorter, 18 weeks or shorter, 16 weeks or shorter, 14 weeks or shorter, or 12 weeks or shorter, and may be determined by a sufficient time to reach the final target dose. For example, when the dose titration is performed using less than 6 mg of efpeglenatide, administration may be performed for 2 weeks, and when the dose titration is performed using 6 mg or more of efpeglenatide, administration may be performed for 2 weeks or 4 weeks, but is not limited thereto.

For example, the method of preventing or treating obesity may further comprise, but is not limited to, a step of administering efpeglenatide to obese patients for 2 weeks to 20 weeks, with sequential dose increments of efpeglenatide ranging from 2 mg to 16 mg, or 2 mg or more and less than 16 mg.

Specifically, the method may further comprise, before the step of administering 8 mg to 18 mg of efpeglenatide, sequentially (a) a step of administering 2 mg of efpeglenatide once a week for 2 weeks, (b) a step of administering 4 mg of efpeglenatide once a week for 2 weeks, (c) a step of administering 6 mg of efpeglenatide once a week for 4 weeks, (d) a step of administering 8 mg of efpeglenatide once a week for 4 weeks, (e) a step of administering 10 mg of efpeglenatide once a week for 4 weeks, (f) a step of administering 12 mg of efpeglenatide once a week for 4 weeks, (g) a step of administering 14 mg of efpeglenatide once a week for 4 weeks, and (h) a step of administering 16 mg of efpeglenatide once a week for 4 weeks, and depending on the patient's drug tolerance, the method may comprise only some steps of the (c) to (h) steps, but is not limited thereto.

For another example, the method of preventing or treating obesity may further comprise, but is not limited to, a step of administering efpeglenatide to obese patients for 2 weeks to 12 weeks, with sequential dose increments of efpeglenatide ranging from 2 mg to 8 mg, or 2 mg or more and less than 8 mg.

Specifically, the method may further comprise, before the step of administering 8 mg to 10 mg of efpeglenatide, sequentially (a) a step of administering 2 mg of efpeglenatide once a week for 2 weeks, (b) a step of administering 4 mg of efpeglenatide once a week for 2 weeks, (c) a step of administering 6 mg of efpeglenatide once a week for 2 weeks or 4 weeks, and (d) a step of administering 8 mg of efpeglenatide once a week for 2 weeks or 4 weeks, but is not limited thereto.

The efpeglenatide, dose titration period, pharmaceutical composition, obesity, and administration are as previously described.

Still another aspect of the present disclosure provides use of the composition comprising efpeglenatide in the preparation of pharmaceutical agents for preventing or treating obesity.

Specifically, efpeglenatide may be administered parenterally (particularly, subcutaneously) to obese patients once a week at a dose of 8 mg to 18 mg, 8 mg to 14 mg, or 8 mg to 10 mg, but is not limited thereto.

The obese patients may be patients who have undergone the dose titration period, but are not limited thereto.

The efpeglenatide, dose titration period, pharmaceutical composition, obesity, and administration are as previously described.

Still another aspect of the present disclosure provides a preparation comprising efpeglenatide for preventing or treating obesity.

Specifically, the preparation may be administered parenterally (particularly, subcutaneously) to obese patients once a week at a dose of 8 mg to 18 mg, 8 mg to 14 mg, or 8 mg to 10 mg, but is not limited thereto.

The patients may be patients who have undergone the dose titration period, but are not limited thereto.

The efpeglenatide, dose titration period, pharmaceutical composition, obesity, and administration are as previously described.

Hereinafter, the present disclosure will be described in more detail with reference to the following exemplary embodiments. However, the following exemplary embodiments are only for illustrating the present disclosure, and the scope of the present disclosure is not intended to be limited thereby.

### Example 1: Structure and Preparation of Efpeglenatide

Efpeglenatide comprised in the composition of the present disclosure is a chemical conjugate of an exendin-4 analogue (CA exendin-4) and a human-derived immunoglobulin IgG4 Fc region linked via 3.4 kDa of a propionaldehyde polyethylene glycol linker molecule (see CAS No. 1296200-77-5, US 8476230 B2).

The CA exendin-4 is a synthetic exendin-4 analogue consisting of 39 amino acids, in which a first residue is substituted with (imidazol-4-yl)acetic acid.

The human-derived immunoglobulin IgG4 Fc region is a homodimer (homodimer, SEQ ID NO: 3) with a single disulfide bond between cysteines at position 3 of a monomer (SEQ ID NO: 2) and has a molecular weight of 49.8 kDa. Each monomer consisting of 221 amino acids comprises two disulfide bonds between cysteines at positions 35 and 95, and at positions 141 and 199.

The binding of CA exendin-4 to the immunoglobulin IgG4 Fc region is achieved by forming an amine bond between aldehyde polyethylene glycol (ALD-PEG-ALD) and lysine at position 27 of CA exendin-4, and the N-terminal amino acid group of the human-derived immunoglobulin IgG4 Fc region.

The structure of efpeglenatide is as follows.
Modified exendin-4 HGEGTFTSDL SKQMEEEAVR LFIEWLKNGG PSSGAPPPS 39
hIGHG4 Fc monomer 
PS CPAPEFLGGP SVFLFPPKPK DTLMISRTPE VTCVVVDVSQ 50'
EDPEVQFNWY VDGVEVHNAK TKPREEQFNS TYRVVSVLTV LHQDWLNGKE 100'
YKCKVSNKGL PSSIEKTISK AKGQPREPQV YTLPPSQEEM TKNQVSLTCL 150'
VKGFYPSDIA VEWESNGQPE NNYKTTPPVL DSDGSFFLYS RLTVDKSRWQ 200'
EGNVFSCSVM HEALHNHYTQ KSLSLSLGK 229'
hIGHG4 Fc monomer 
PS CPAPEFLGGP SVFLFPPKPK DTLMISRTPE VTCVVVDVSQ 50"
EDPEVQFNWY VDGVEVHNAK TKPREEQFNS TYRVVSVLTV LHQDWLNGKE 100"
YKCKVSNKGL PSSIEKTISK AKGQPREPQV YTLPPSQEEM TKNQVSLTCL 150"
VKGFYPSDIA VEWESNGQPE NNYKTTPPVL DSDGSFFLYS RLTVDKSRWQ 200"
EGNVFSCSVM HEALHNHYTQ KSLSLSLGK 229"
Disulfide bridges location

| | | | | |
|---|---|---|---|---|
| 11'-11" | 43'-103' | 43"-103" | 149'-207' | 149"-207" |

Modified residues

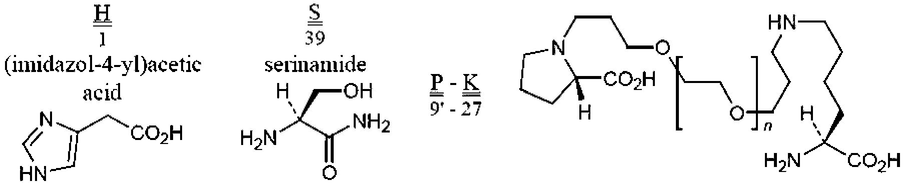

It was intended to examine whether or not a weight loss effect may be obtained in obese patients by administering 8 mg to 18 mg of efpeglenatide once a week, through non-clinical and clinical trials.

### Experimental Example 1: Verification of Weight Loss Effect by Efpeglenatide in Animal Model

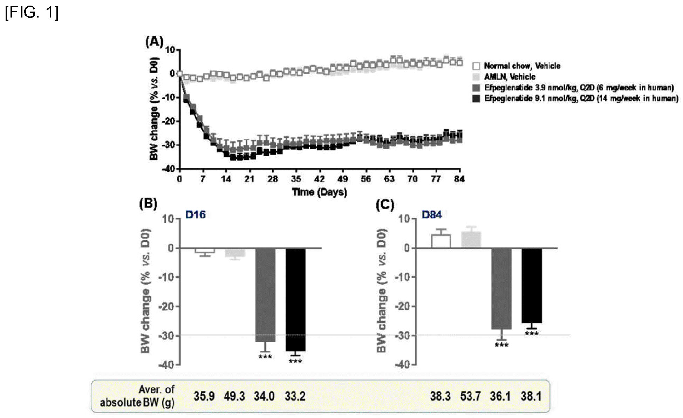

To verify the weight loss effect from high-dose administration of efpeglenatide, 6 mg or 14 mg of the human equivalent dose (HED) of efpeglenatide was administered once a week to obese mice in which obesity was induced by AMLN diet (high fat, high fructose, high cholesterol) (FIG. 1).

At this time, the method of converting the mouse dose into the human dose is as follows.
1. Conversion to molar concentration
2. Reflection of dosing interval - Q2D in mice corresponds to QW in humans, and the dose to be administered for one week was administered once every two days in divided doses, and therefore, the dose administered for one week was calculated by applying 3.5 times
3. Mouse dose (mg/kg) = 12.3 x human dose (mg/kg), upon calculating the human equivalent dose considering the body surface area (m²)
4. Calculated, based on 100 kg of obese patient

As a result, as shown in FIG. 1, the administration of efpeglenatide leads to a gradual weight loss during the initial 2 weeks to 3 weeks, followed by a slight weight gain, which is commonly observed in the weight loss profile of GLP-1 preparations.

The 6 mg and 14 mg administration groups both showed similar weight loss effects, and a weight loss effect of up to 30% was observed on Day 16 (D16). Although body weight slightly increased thereafter, both administration groups maintained normalized body weight until the end of the study.

These results suggest that efpeglenatide is effective in losing the body weight and maintaining the lost body weight, and furthermore, it is understood that the safety of efpeglenatide was verified, as it did not show excessive weight loss below the normal body weight even with the high-dose administration.

A clinical trial was conducted with the administration of 8 mg to 10 mg of efpeglenatide, which showed weight loss effects in animal models as described.

### Experimental Example 2: Verification of Weight Loss Effect by Efpeglenatide in Humans

To examine the weight-loss effect of efpeglenatide in adult obese patients without diabetes, a multicenter, randomized, double-blind, placebo-controlled, parallel-design clinical trial was conducted. The 'mean body weight change rate (%) from baseline at week 40' and the 'proportion (%) of subjects with a weight loss rate of 5% or more from baseline at week 40' of efpeglenatide were examined to demonstrate the superiority of efpeglenatide to placebo, and the safety was comparatively assessed. In addition, the weight-loss effect and safety were observed up to week 64.

### Experimental Example 2-1. Selection of Participants

420 participants in this clinical trial were selected based on the following criteria:
On the first day of the visit (Visit 1),
- Adults aged 18 years or older
- Satisfying any of the obesity criteria below
   - Those with BMI ≥ 30 kg/m²
   - Those with 27 kg/m² ≤ BMI < 30 kg/m² and at least one or more risk factors or comorbidities

Here, risk factors or comorbidities refer to the following:
i) hypertension: taking medication for hypertension or having sitSBP ≥ 140 mmHg or sitDBP ≥ 90 mmHg
ii) dyslipidemia: taking medication for dyslipidemia or having total cholesterol ≥ 240mg/dL or LDL-C ≥ 160mg/dL or TG ≥ 200 mg/dL or HDL-C < 40 mg/dL
iii) sleep apnea
iv) cardio-cerebrovascular diseases: ischemic cardio-cerebrovascular diseases, heart failure of NYHA Class I~III, etc.

However, participants who were found to have diabetes (type 1, type 2, etc.) or HbA1c ≥ 6.5% were excluded.

### Experimental Example 2-2. Administration Drugs and Administration method

### (1) Test drugs

① Prefilled syringe containing 2 mg/0.5 mL of efpeglenatide
② Prefilled syringe containing 4 mg/0.5 mL of efpeglenatide
③ Prefilled syringe containing 6 mg/0.5 mL of efpeglenatide
④ Prefilled syringe containing 8 mg/0.5 mL of efpeglenatide
⑤ Prefilled syringe containing 10 mg/0.5 mL of efpeglenatide

### (2) Placebo

Prefilled syringe comprising 0.5 mL of efpeglenatide placebo

### (3) Administration method

Participants selected in Experimental Example 2-1 were randomly assigned, and the randomly assigned subjects were administered the full amount of assigned efpeglenatide or placebo contained in the prefilled syringe, 0.5 mL per injection, once a week during the clinical trial period.

### Experimental Example 2-3. Determination of Dosage

It was found that overall drug tolerance was good at doses up to 6 mg once a week and up to 8 mg once every two weeks in a 20-week, randomized, double-blind, placebo-controlled, parallel-group, phase 2b clinical trial to assess the safety and efficacy of efpeglenatide on the body weight in adult obese patients without diabetes.

However, as the dose of the previously approved GLP-1 analogue drugs of the same series increased, the effect (weight loss) tended to increase (Liraglutide 1.8 mg vs. 3.0 mg about 1.7 times, Semaglutide 1 mg vs. 2.4 mg about 2.4 times), and as efpeglenatide was also found to have the pattern of high weight loss effect at high doses, as compared to low doses, in adult obese patients, the target dose was set to 10 mg, which is about 1.7 times that of 6 mg, for additional weight loss effect.

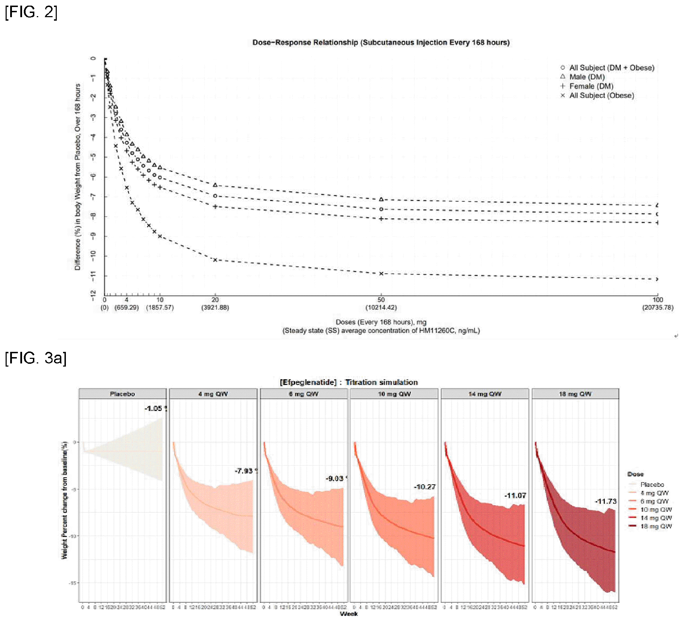

As a result of estimating the maximum effect size based on the weight loss rate through population PK/PD simulation, the maximum effect (Eₘₐₓ) of efpeglenatide in obese patients at 60 weeks with a once-weekly administration regimen was estimated to be about -11%, and the dose of 10 mg was expected to show about 80% or more of the maximum effect (FIG. 2).

To explain the relationship between the efpeglenatide concentration and the long-term body weight changes, pharmacodynamic modeling (a turn-over model in which the concentration inhibits the response) was performed. In detail, the simulation was conducted in such a way that 50 subjects each for the target doses of 4 mg, 6 mg, 10 mg, 14 mg, and 18 mg QW were administered by applying dose titration, which was repeated 100 times, and the body weight baseline of the subjects was set based on the results of the existing phase 2 clinical trial. As a result, the predicted body weight loss rates according to the administration dose of efpeglenatide were -7.93%, -9.03%, -10.27%, -11.07%, and -11.73% for the doses of 4 mg, 6 mg, 10 mg, 14 mg, and 18 mg, respectively (FIG. 3A).

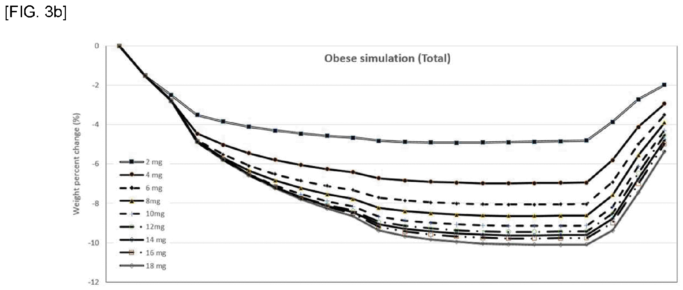

In addition, a population pharmacokinetic-pharmacodynamic (PK-PD) simulation was performed to estimate the body weight loss rate from the baseline when the target dose was reached at 2-week intervals for each administration dose in obese patients (FIG. 3B). In detail, in order to investigate the body weight loss rate at each time point, for target doses of 2 mg, 4 mg, 6 mg, 8 mg, 10 mg, 12 mg, 14 mg, 16 mg, and 18 mg QW, the dose was increased by increments of 2 mg at 2-week intervals to reach the target dose, and repeated administration once a week until week 59. The results of predicting the trend of the weight loss rate during the washout period (week 72) are shown in Table 1.

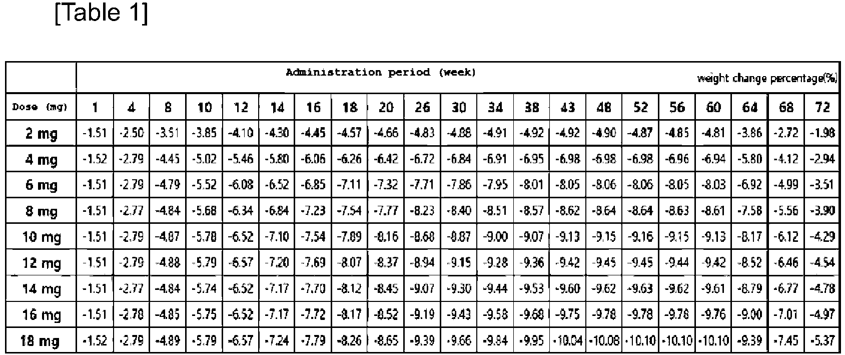

### Experimental Example 2-4. Determination of Administration Cycle

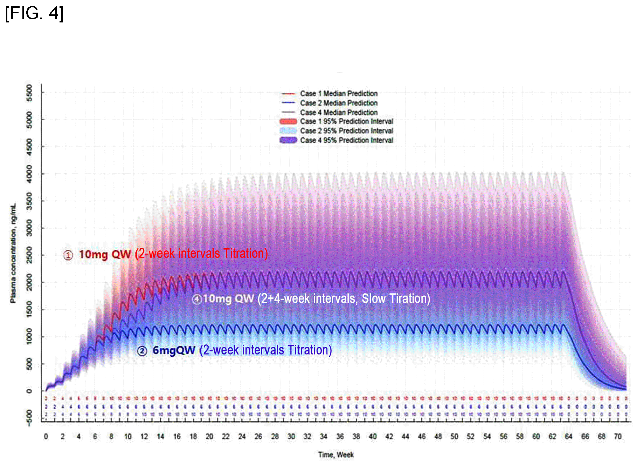

Since GLP-1 analogue drugs tend to have higher gastrointestinal adverse reactions at the beginning of administration and at higher doses, a slow titration period was introduced in this clinical trial as a strategy to reduce the safety risk at the initial exposure of 10 mg (FIG. 4).

In other words, until reaching 6 mg of efpeglenatide, 2 mg and 4 mg were each administered for 2 weeks, and until reaching 10 mg, 6 mg and 8 mg were each administered for 4 weeks, thereby increasing the dose more slowly to reach the target dose.

When comparing the cumulative exposure which was predicted through population pharmacokinetic modeling, a tendency of about 16% to 21% decrease in systemic exposure was observed when increasing the dose at 2+4-week intervals (slow titration), as compared to 2-week intervals. This may alleviate the initial adverse reactions.

**[Table 2]**

| **Comparison of cumulative exposure according to dose increment (2 weeks vs. 2+4 weeks)** | | | | | | | |
|---|---|---|---|---|---|---|---|
| **Time point** | **②6mg QW (2-week Interval titration)** | | **①10mg QW (2-week Interval titration)** | | **④10mg QW (2+4 week Interval slow titration)** | | **AUC' (① *vs* ④ reduction %)** |
| | **Cₘₐₓ (ng/mL)** | **AUC (h*ng/mL)** | **Cₘₐₓ (ng/mL)** | **AUC (h*ng/mL)** | **Cₘₐₓ (ng/mL)** | **AUC (h*ng/mL)** | |
| **4wks** | 481.18 | 75076.64 | 481.97 | 75196.38 | 481.97 | 75196.38 | - |
| **8wks** | 1054.38 | 166718.73 | 1261.39 | 199471.99 | 1055.99 | 166931.39 | -16.31 |
| **12wks** | 1218.15 | 192893.53 | 1975.55 | 313800.60 | 1561.34 | 247645.96 | -21.08 |

| | | | | | | | |
|---|---|---|---|---|---|---|---|
| ^{†}Cumulative exposure up to the corresponding timepoint (Weekly dosing population PK^{‡} simulation from Asan's modeling, 2023) ^{‡}Data sets included in modeling: HM-EXC-102, 201, 202, 203, 204, 205, AMPLITUTE-M | | | | | | | |

Additionally, the difference in the probability of major gastrointestinal adverse reactions based on dose titration intervals (no dose titration, 2-week intervals, or 4-week intervals) was investigated through clinical trials.

Specifically, when comparing the major gastrointestinal adverse reactions between the once-weekly 6 mg administration group (no dose titration) in the phase 2 clinical trial (HM-EXC-205) targeting adult obese patients and the once-weekly 6 mg administration group (dose titration with increments of 2 mg at 2-week intervals) from a sub-analysis of obese patients with a BMI of 27 or higher in the phase 3 clinical trial (AMPLITUDE-M) targeting type 2 diabetes patients, it was confirmed that the safety outcomes in phase 3 in which dose titration was performed were better (Table 3).

**[Table 3]**

| | **HM-EXC-205(at week 20)** | | | **AMPLITUDE-M (BMI>=27) (at week 56)** | | |
|---|---|---|---|---|---|---|
| | **Placebo (n=60)** | **6 mg QW (n=59)** | **8 mg Q2W (n=58)** | **Placebo (n=92)** | **4 mg QW (n=88)** | **6 mg QW (n=89)** |
| Gastrointestinali disorders † | 28(46.7%) ,78 | 49(83.1%), 220 | 44(75.9%), 206 | 26(28.26%) , 57 | 40(45.45%) ,100 | 54(60.67%) ,125 |
| Nausea | 11 (18.3%), 14 | 35 (59.3%), 75 | 36 (62.1%), 117 | 2 (2.17%), 3 | 12 (13.64%), 20 | 18 (20.22%), 24 |
| Diarrhoea | 12 (20.0%), 23 | 12 (20.3%), 17 | 16 (27.6%), 36 | 8 (8.87%), 16 | 14 (15.51%), 16 | 21 (23.60%), 27 |
| Vomiting | 4 (6.7%), 4 | 13 (22.0%), 37 | 19 (32.8%), 28 | 0 (0.0%), 0 | 5 (5.68%), 5 | 7 (7.87%), 0 |
| Dyspepsia | 2 (3.3%), 2 | 16 (27.1%), 25 | 15 (25.9%), 20 | 2 (2.17%), 2 | 7 (7.05%), 10 | 11 (12.36%), 16 |
| Constipation | 5 (8.3%), 5 | 12 (20.3%), 15 | 12 (20.7%), 10 | 6 (6.52%), 6 | 12 (13.64%), 20 | 14 (15.73%), 15 |
| Abdominal pain | 2 (3.3%), 2 | 11 (18.6%), 16 | 8 (13.8%), 15 | 4 (4.35%), 4 | 1 (1.14%), 1 | 1 (1.12%), 1 |
| Abdominal pain upper | 3 (5.0%), 0 | 6 (10.2%), 6 | 9 (15.5%), 17 | 2 (2.17%), 2 | 2 (2.27%), 3 | 2 (2.25%), 2 |
| Abdominal distension | 1 (1.7%), 1 | 5 (8.5%), 7 | 8 (13.8%), 9 | 2 (2.17%), 2 | 3 (3.41%), 3 | 6 (6.74%), 7 |
| Gastrooesopha geal ref lux disease | 2 (3.3%), 2 | 2 (3.4%), 2 | 3 (5.2%), 4 | 0 (0.00%), 0 | 4 (4.55%), 4 | 4 (4.49%), 4 |
| Any TEAE Leading to Discontinuation | 4 (6.7%), 5 | 11 (18.6%), 41 | 10 (17.2%), 36 | 7 (7.6%) | 7 (8.0%) | 12 (13.5%) |
| ^{†}Based on adverse reactions that occurred in 5% or more of all subjects administered efpeglenatide in each clinical trial. | | | | | | |

Additionally, when comparing the results of a sub-analysis of obese patients with a BMI of 27 or higher in the phase 3 clinical trial (AMPLITUDE-M) targeting type 2 diabetes patients to the results of another phase 3 clinical trial (AMPLITUDE-O study, type 2 diabetes patients), it was confirmed that even at the same target dose (6 mg), when the dose increments were performed slowly, i.e., when the dose increment interval was 4 weeks, the probability of major gastrointestinal adverse reactions may be lower than when the dose increment interval was 2 weeks (Table 4, probability of Nausea & Vomiting: 12.3% & 5.5% for 4-week interval titration vs. 20.22% & 7.87% for 2-week interval titration).

**[Table 4]**

| **Study & Titration scheme** | **AMPLITUDE-O¹⁾** | | | **AMPLITUDE-M²⁾ (BMI>=27 subgroup)** | | |
|---|---|---|---|---|---|---|
| | **Dose increase every 4 weeks (2-2-2-2-4-4-4-4-6mg .... )** | | | **Dose increase every 2 weeks (2-2-4-4-6mg ....)** | | |
| **# pts (%), #AEs** | **Placebo (n=1355)** | **4 mg QW (n=1360)** | **6 mg QW (n=1358)** | **6 mg QW (n=89)** | **4 mg QW (n=88)** | **Placebo (n=92)** |
| GI disorders | 237(17.5%) | 478(35.1 %) | 480(35.3 %) | 54(60.67%), 1 25 | 40(45.45%), 1 00 | 26(28.26%), 57 |
| Nausea | 51(3.8%) | 197(14.5 %) | 167(12.3 %) | 18(20.22%), 24 | 12(13.64%). 20 | 2(2.17%), 3 |
| Vomiting | 19(1.4%) | 88(6.5%) | 75(5.5%) | 7(7.87%), 9 | 5(5.68%), 5 | 0(0.0%), 0 |
| Diarrhoea | 67(4.0%) | 117(8.6% ) | 118(8.7% ) | 21(23.60%), 27 | 14(15.91%), 16 | 8(8.87%), 16 |
| Constipation | 33(2.4%) | 64(4.7%) | 56(4.1%) | 14(15.73%), 15 | 12(13.64%), 20 | 6(6.52%), 6 |
| Dyspepsia | 9(0.7%) | 57(4.2%) | 53(3.0%) | 11(12.36%), 16 | 7(7.95%), 10 | 2(2.17%), 2 |
| Abdominal pain | 13(1.0%) | 30(2.2%) | 34(2.5%) | 1(1.12%), 1 | 1(1.14%), 1 | 4(4.35%), 4 |
| Abdominal pain upper | 17(1.3%) | 19(1.4%) | 23(1.7%) | 2(2.25%), 2 | 2(2.27%), 3 | 2(2.17%), 2 |
| Abdominal distension | 7(0.5%) | 17(1.3%) | 25(1.8%) | 6(6.74%), 7 | 3(3.41%), 3 | 2(2.17%), 2 |
| GERD | 8(0.6%) | 31(2.3%) | 33(2.4%) | 4(4.40%), 4 | 4(4.55%), 4 | 0(0.00%), 0 |
| Gastritis | 17(1.3%) | 20(1.5%) | 31(2.3%) | 0(0.00%), 0 | 0(0.00%), 0 | 1(1.09%), 1 |
| Eructation | 1(<0.1%) | 22(1.6%) | 26(1.9%) | 1(1.12%), 1 | 3(3.41%), 3 | 1(1.09%), 1 |
| Flatulence | 4(0.3%) | 16(1.2%) | 29(2.1%) | 6(6.74%), 6 | 2(2.27%), 2 | 1(1.09%), 1 |
| ¹⁾T2DM patients with high CV risk (median 1.81 years), ²⁾T2DM patients (administered for 56 weeks) | | | | | | |

Based on these results, a dose titration method was established, where when the target dose is a high dose, less than 6 mg is administered for 2 weeks and 6 mg or more is administered for 4 weeks.

Furthermore, since nausea and vomiting are expected as the main adverse reactions to efpeglenatide administration, a population PK/AE simulation (weekly dosing) was conducted focusing on the aforementioned adverse reactions. The probability of adverse reactions predicted by the corresponding population PK/AE model was 62.7% and 76.3% (at 18 weeks) when the target doses of 6 mg and 10 mg were administered without the dose titration period, respectively. It was confirmed that this predicts a similar or slightly higher level, compared to the adverse reaction rate for 6 mg observed in a practical obesity clinical trial (20 weeks, 59.3%).

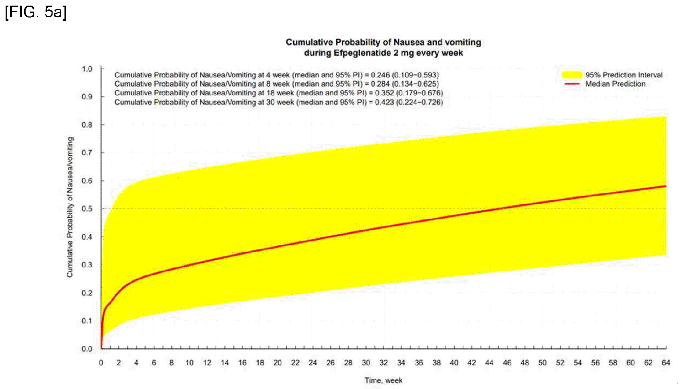

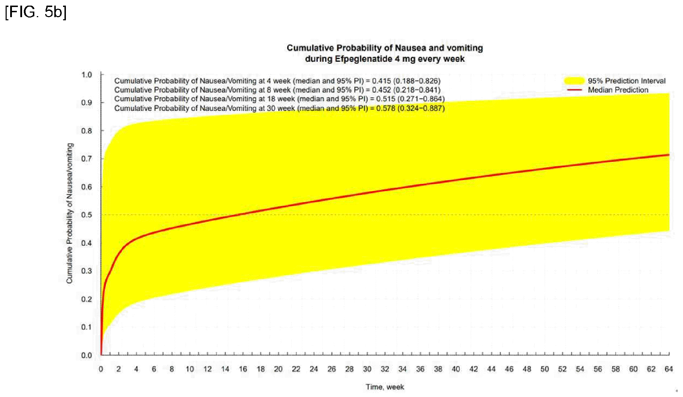

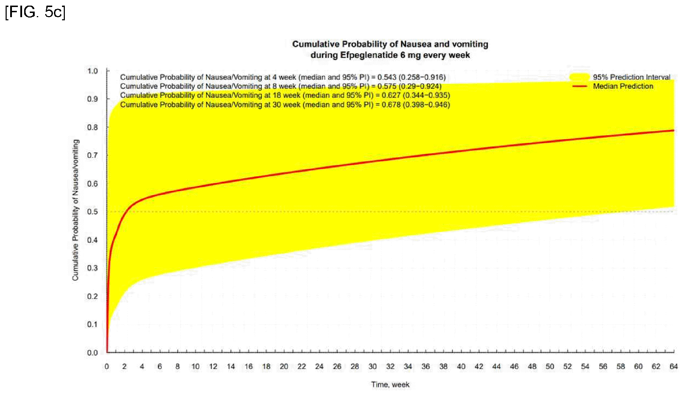

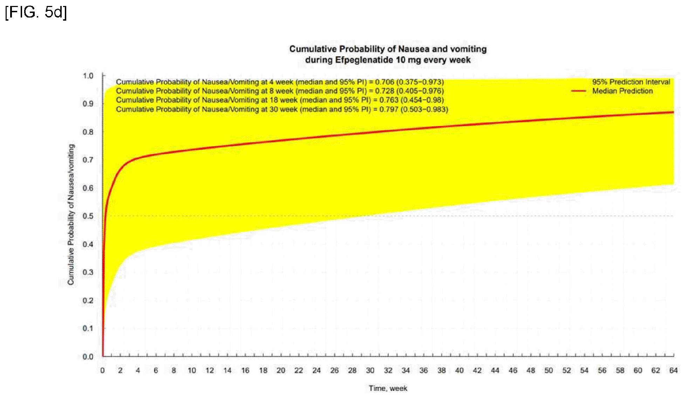

The probability of adverse reactions predicted at 4 weeks, 8 weeks, 12 weeks, and 38 weeks was about 17%, about 23%, about 26%, and about 30%, respectively, which was lower than the probability (42% to 58%) of adverse reactions predicted at the maintenance doses of 2 mg, 4 mg, 6 mg, and 10 mg under conditions without the dose titration period (FIG. 5).

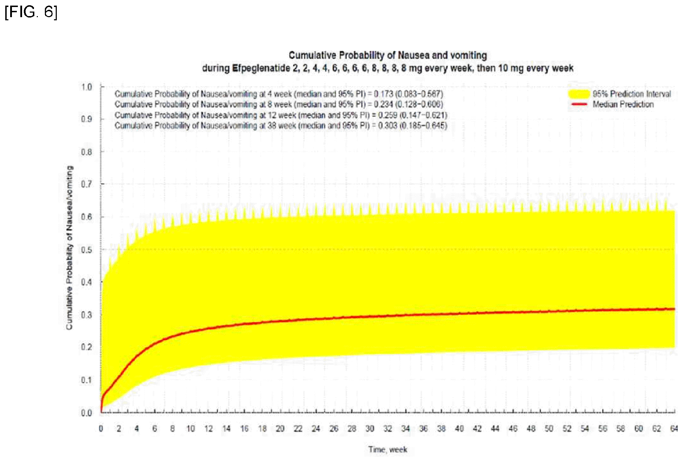

Accordingly, the results of predicting the cumulative probability of adverse reactions when reaching and maintaining the target dose of 10 mg after dose titration (2+4 weeks) using the corresponding model are shown in FIG. 6.

Specifically, the probability of adverse reactions rapidly increased until the initial 4-week point, but gradually stabilized thereafter.

### Experimental Example 2-5. Test Method

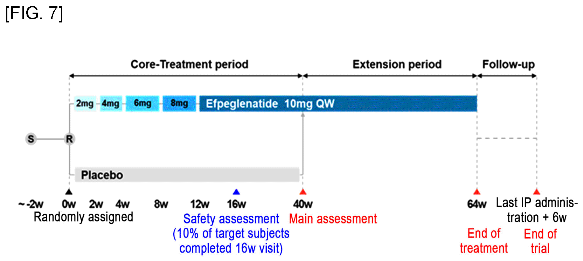

The clinical trial was conducted in the same manner as in FIG. 7.

In brief, participants selected according to the criteria in Experimental Example 2-1 were classified according to body mass index (27 kg/m² ≤ BMI < 30 kg/m², BMI ≥ 30 kg/m²) and gender, and randomly assigned to a test group and a control group at a ratio of 2:1.

The period of about 40 weeks after randomization was a core-treatment period, which is a period for comparative assessment with a placebo. The test group started with 2 mg of efpeglenatide, which was increased by 1 step (2 mg) (Titration), and then gradually increased at 2-week intervals until reaching 6 mg, and then at 4-week intervals until reaching 10 mg. If possible, 10 mg of the target dose of efpeglenatide should be reached by week 12, and then maintained at the corresponding dose. The control group was administered the same amount of placebo as the amount of the test group at all time points.

The period of about 24 weeks, up to week 64 after the core-treatment period, was an extension period. The test group maintained the existing dose, which was administered during the core-treatment period, until the end of treatment, and the control group switched from placebo to efpeglenatide at week 40. At this time, the control group started with 2 mg of efpeglenatide, which was increased by 1 step (2 mg) (Titration), and then gradually increased at 2-week intervals until reaching 6 mg, and then at 4-week intervals until reaching 10 mg. Once 10 mg of the target dose of efpeglenatide was reached, the corresponding dose was maintained until the end of treatment.

Finally, a trial termination visit was conducted 6 weeks after the last IP administration to assess the efficacy and safety during the washout state of the clinical trial drug and to conclude the clinical trial.

### Experimental Example 2-6. Efficacy Assessment Criteria

The efficacy of efpeglenatide administration in participants was assessed, based on the following items:
(1) Primary efficacy assessment variables
   - Change in body weight at 40 weeks from baseline
   - Proportion of subjects with a weight loss rate of 5% or more at 40 weeks from baseline
(2) Secondary efficacy assessment variables
   - Change in body weight at 24 weeks, 64 weeks from baseline
   - Proportion of subjects with a weight loss rate of 5% or more at 24 weeks, 64 weeks from baseline
   - Proportion of subjects with a weight loss rate of 10% or more at 24 weeks, 40 weeks, 64 weeks from baseline
   - Change in body weight at 24 weeks, 40 weeks, 64 weeks from baseline
   - Change in BMI at 24 weeks, 40 weeks, 64 weeks from baseline
   - Change in waist circumference at 24 weeks, 40 weeks, 64 weeks from baseline
   - Change in glucose metabolism indices at 24 weeks, 40 weeks, 64 weeks from baseline: HbA1c, FPG, Fasting insulin (Fasting insulin was presented as a percentage change from baseline)
   - Change in lipids at 24 weeks, 40 weeks, 64 weeks from baseline: Total cholesterol, LDL-C, HDL-C, TG, non-HDL-C
   - Change in blood pressure at 24 weeks, 40 weeks, 64 weeks from baseline: sitSBP, sitDBP
   - Change in IWQoL-Lite-CT (Physical function domain) at 24 weeks, 40 weeks, 64 weeks from baseline

### Experimental Example 2-7. Safety and Exploratory Assessment Criteria

Safety and exploratory assessments of efpeglenatide administration in participants were performed, using the following items.
(1) Exploratory Assessments
   - Body composition assessment (DEXA, BIA): Body fat mass, lean body mass, visceral fat mass, etc.
   - Immunogenicity and pharmacokinetic assessment
   - Hepatic steatosis assessment, etc.
(2) Safety assessment: Adverse reactions, vital signs, clinical laboratory tests, physical examination, electrocardiogram, mental health assessment (PHQ-9, C-SSRS)

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A pharmaceutical composition for preventing or treating obesity, the pharmaceutical composition comprising efpeglenatide as an active ingredient, wherein 8 mg to 18 mg of efpeglenatide is parenterally administered to obese patients once a week.

2. The pharmaceutical composition of claim 1, wherein 8 mg to 10 mg of efpeglenatide is parenterally administered to obese patients once a week.

3. The pharmaceutical composition of claim 1, wherein the obese patients have a body mass index (BMI) of 25 kg/m² or higher.

4. The pharmaceutical composition of claim 1, wherein the obese patients do not have diabetes.

5. The pharmaceutical composition of claim 3, wherein the obese patients have
(a) a body mass index (BMI) of 30 kg/m² or higher, or
(b) a BMI of 27 kg/m² or higher, and lower than 30 kg/m² while having one or more risk factors or comorbidities.

6. The pharmaceutical composition of claim 5, wherein the patients having risk factors or comorbidities have any one or more diseases of hypertension, dyslipidemia, sleep apnea, and cardio-cerebrovascular diseases.

7. The pharmaceutical composition of any one of claims 1 to 6, wherein the pharmaceutical composition is used in dosing regimens for preventing or treating obesity.

8. The pharmaceutical composition of any one of claims 1 to 6, wherein the obese patients have undergone a dose titration period in which 2 mg to 16 mg of efpeglenatide is administered for 2 weeks to 12 weeks.

9. The pharmaceutical composition of claim 8, wherein the dose titration is performed by sequentially administering 2 mg of efpeglenatide once a week for 2 weeks, 4 mg of efpeglenatide once a week for 2 weeks, 6 mg of efpeglenatide once a week for 4 weeks, and 8 mg of efpeglenatide once a week for 4 weeks.

10. The pharmaceutical composition of claim 8, wherein when the titration is performed with a dose of 2 mg or more and less than 6 mg, administration is performed once a week for 2 weeks, and when the titration is performed with a dose of 6 mg or more and 16 mg or less, administration is performed once a week for 4 weeks, sequentially.

11. A method of preventing or treating obesity, comprising a step of parenterally administering 8 mg to 18 mg of a pharmaceutical composition comprising efpeglenatide as an active ingredient to obese patients once a week.

12. The method of claim 11, further comprising a dose titration step in which 2 mg to 16 mg of efpeglenatide is administered for 2 weeks to 12 weeks before parenterally administering 8 mg to 18 mg to obese patients once a week.

13. Use of a composition comprising efpeglenatide for preparing a medicament for preventing or treating obesity,
wherein 8 mg to 18 mg of efpeglenatide is parenterally administered to obese patients once a week.
